# EUROPEAN PATENT APPLICATION

(11) **EP 4 374 867 A1**
(43) Date of publication of application: **29.05.2024**
(21) Application number: 22208886.6
(22) Date of filing: 22.11.2022
(51) Int. Cl.: A61K 36/88, A61K 31/00, A61P 35/00

(54) **WATER SOLUBLE POLYSACHARIDE EXTRACTION METHOD FROM SAFFRON CORMS AND ITS APPLICATION**

(71) Applicant: Lietuvos sveikatos mokslu universitetas, 44307 Kaunas (LT)
(72) Inventor: Petrikaité, Vilma, 44307 Kaunas (LT); Ivanauskas, Liudas, 44307 Kaunas (LT); Mykhailenko, Olga, 61002 Kharkiv (UA); Georgiyants, Victoriya, 61002 Kharkiv (UA)
(74) Representative: Zaboliene, Reda

(57) **Abstract**

The present invention relates to the method of preparation of water-soluble extract of polysaccharide complex from *Crocus sativus* corms and their use in medicine, in particularly for the treatment of cancer. The water-soluble polysaccharide extract is obtained by selecting raw *Crocus sativus* corms, crushing them and macerating them in room temperature water (18-20°C) for 24 hours. Water-soluble polysaccharides are then extracted with water and precipitated. The resulting polysaccharide extract is dried and comminuted to a powder. The resulting water-soluble polysaccharide extract is intended for use in prophylaxis or treatment of cancer.

## Description

### TECHNICAL FIELD

This invention is related to the technical field of polysaccharide extraction, in particular, to water-soluble polysaccharide extraction method from *Crocus sativus* corms and its application in medicine.

### BACKGROUND

*Crocus sativus* is most often used in food production as a colourant and flavourant, however, currently, its healing properties are receiving an increasing interest. There is an increasing amount of data about the use of saffron in folk medicine in the treatment of cancer of various etymologies, eye diseases and its use as an antiviral and toning preparation.

The corms are crushed, flattened at the base to a diameter of about 4.5 to 5.5 cm and covered with several fibrous and cancellated covers. Corms have one or two main buds at the apex and approximately (depending on size) 4-5 or more secondary buds arranged irregularly in a spiral shape (Gresta et al., 2008).

*Crocus sativus* belongs to the *Crocus* genus of the abundant *Iridaceae* family. Since 2015 Saffron cultivation for food production was started in the Kherson region (Ukraine). Dried stigmas of *Crocus sativus* flowers are used as a spice of the so-called saffron. These spices are well known for their colour, bitterness, aroma and are the most expensive in the world. In order to extract 1 kg of saffron, many by-products are obtained: about 350 kg of perianth leaves, 1,500 kg of leaves and hundreds of corms, which are unfit for cultivation due to physical, biological damage or other reasons and are discarded. However, these unused by-products can be used to extract commercially valuable polysaccharides.

There is a known method described in patent application CN106589152A according to which polysaccharides are extracted from *Xinjiang Araceae* saffron corms by macerating them in water and precipitating released polysaccharides using ethanol. The invention described herein, however, uses no toxic reagents such as chloroform or n-butanol.

In Chinese patent application CN109320628A, maceration in water and precipitation of polysaccharides using alcohol is also used to extract polysaccharides from *Crocus sativus.* The difference of the invention described herein is that it involves fewer steps and is specifically optimised for extraction from saffron corms.

Patent application UA142213U also describes a method to extract polysaccharides from saffron corms using maceration in water. The difference is that in the method described herein maceration is performed at room temperature (18-20°C) and less water is used.

The invention described herein discloses a method to obtain a high yield, high purity and reduce the extraction time of water-soluble polysaccharides from saffron corms.

### SUMMARY OF THE INVENTION

In order to solve the above-mentioned problems and the emission of by-products from the production of saffron, the present invention provides a method for extracting water-soluble polysaccharides from *Crocus sativus* corms. Water-soluble polysaccharides from *Crocus sativus* corms are obtained using the following steps:
1) crushing and macerating saffron corms in water at room temperature three times for 24 hours;
2) separating the liquids from the solids, where the solids are filtered out and the liquid component is collected to obtain a water-dissolved saffron corm extract;
3) precipitating the polysaccharides from the saffron corm extract using ethanol in order to remove the active substances from the remaining liquid medium;
4) drying the precipitated material in an oven at 60-65° C until the precipitated material reaches a constant weight. The precipitated material is then grounded in a mortar to obtain a powdered material.

The saffron corms are crushed to a particle size passing through a 0.5-1 mm sieve. Preferably, the maceration is performed using a 1 g: 4 ml ratio of crushed saffron corms to water and the extraction temperature is between 18 and 20°C.

Preferably, the saffron corm extract is evaporated to a volume of 40 ml on a rotary evaporator at 80°C.

The prepared dry polysaccharide extract is water soluble and substantially comprises polysaccharides.

All tested dry extracts of water-soluble polysaccharides from saffron corms have shown anti-cancer activity for cancer, preferably for human triple-negative breast cancer and human melanoma.

### BRIEF DESCRIPTION OF THE DRAWINGS

**Fig. 1****.** Anticancer activity, expressed as ECso values in IGR39 and MDA-MB231 cell lines, n = 3.
**Fig. 2****.** Comparison of extract activity 2 and 5 in IGR39 and MDA-MB231 cell lines. Experimental data are shown as points and theoretical fit data (according to Hill's equation) as lines.

### DETAILED DESCRIPTION

The present invention provides a process for the extraction of polysaccharides from saffron corms which is carried out in the following way.

Raw materials were used for the study, which, depending on the vegetation cycle of the plant, were at the beginning of the stage of leaf bud emergence and differentiation in corms. In this biological cycle, transition from dormancy to active metabolism occurs due to an increase in the amount of sugars and other metabolites involved in the tricarboxylic acid, glycolic acid, amino acid, and fatty acid cycle. According to the analysis in the literature, several phenolic acids were found in *Crocus* corms, including caffeic acid, cinnamic acid, ferulic acid, p-coumaric acid, gallic acid, p-hydroxybenzoic acid, gentisic acid, salicylic acid, but in small amounts; the triterpenoid saponins azafrine 1 and azafrine 2 (Rubio-Moraga et al., 2011); and the new glycoconjugate (Fernandez et al., 2000). Because the composition of phenolic compounds in corms is not rich, this invention focuses on the polysaccharide part of the raw material. Polysaccharides (glycans) are natural polymeric carbohydrates whose molecules are made up of monosaccharide residues linked by glycosidic bonds. Numerous biological effects of plant polysaccharides have been identified, such as anti-hepatic, anti-cancer, antiviral, hypoglycemic, anticoagulant, antioxidant, and immunoregulatory effects. Therefore, their extraction and exploration are very promising and necessary. The separation method provided herein meets two main requirements: first, it is the most efficient way to separate low molecular weight materials, with negligible losses at all stages of production; second, only minimal changes occur in the polysaccharide complex at all stages. This method avoids using aggressive, toxic chemical reagents, enzymes and, accordingly, does not allow the destruction of polysaccharides.

**The plant.** *Crocus sativus* L. corms were harvested from a plantation in Lyubimivka village, Kherson region (Ukraine) in July 2019. Before the start of the experiment, the corms were stored in a refrigerator at 3°C for two months. Corms are well-cleaned of last year's tubers, old scales, old sticky leaves, sprouts. Fresh raw materials (not dried) are used for analysis. Shredding is done mechanically.

**Extraction.** To obtain water-soluble polysaccharides, a sample of corms is ground to a particle size passing through a sieve with a diameter of 0.5-1 mm. About 40 g (accurately weighed) of the chopped corms are placed in a 250 ml graduated flask, to which 160 ml of purified room temperature (18 to 20°C) water is also added. The sample is left to macerate for 24 hours, not in direct sunlight. This extraction method is repeated by adding 100 ml of water under the same conditions. The extraction repeated 2 times is sufficient for maximum extraction of water-soluble polysaccharides from saffron corns. The extracts are combined and used for further analysis. The process of precipitating polysaccharides in the extracts is carried out as described below.

**Precipitation.** The saffron corm extract is filtered through a 3-layer gauze with a cotton swab placed in a glass funnel and evaporated on a rotary evaporator ("Heidolph 2 WB eco, Laborata400 efficient", Germany) at 80°C to 40 ml of extract. The precipitation is carried out with a triple amount of 95% ethanol in a cylinder, shaking vigorously to precipitate water-soluble polysaccharides within 20 minutes. The contents of the flask are then filtered using a Buchner funnel through a pre-dried and weighed ashless paper filter placed in a POR 16 glass 40 mm diameter filter under vacuum at a residual pressure of 0.4-0.8 atm. The filtered cake is washed successively with 15 ml of a 95% solution of ethanol in purified water (3:1), 10 ml of a mixture of ethyl acetate and 95% ethanol (1: 1). The precipitation with the triple amount of ethanol provides water-soluble polysaccharide extract which substantially comprises polysaccharides.

**Drying.** The filter cake is first air-dried, then the extract obtained is carefully transferred to a Petri dish and dried in a drying oven at 60 to 65°C until a constant weight of light pink to light grey tasteless and odourless extract is obtained. Also, the dry extract is grounded in a mortar to a powder.

The yield of the dry prepared product is 5.71% (or 2.34 g).

### ANTI-CANCER ACTIVITY

**Cell culture.** The human melanoma cancer cell line IGR39 and the human triple-negative breast cancer cell line MDA-MB-231 were grown in DMEM Glutamax medium (Gibco, Carlsbad, CA, USA) containing a mixture of 10% fetal calf serum and 1% antibiotics (10,000 U/ml of penicillin and 10 mg/ ml of streptomycin; Gibco).

All cells were incubated at 37°C in a humidified atmosphere containing 5% CO₂ until the 20th passage.

**Cell viability study.** Cell viability after exposure to the dry extract of water-soluble polysaccharides from Ukrainian corms of *Crocus sativus* L. was assessed using the MTT (Sigma-Aldrich Co.) reduction method.

IGR39 and MDA-MB-231 cells were seeded (3000 and 5000 cells / well, respectively) in a 96-well plate and incubated overnight at 37°C in a humidified 5% CO₂ atmosphere. Cells were exposed to various test extracts (1 mg / ml to 15.56 µg / ml). After 72 hours of incubation with the extracts, 20 µl of MTT (5 mg / ml) was added to each well and incubated for 3-4 hours under the same conditions. The supernatant was removed and 100 µl of DMSO was added. Absorbance was measured at 570 nm and 630 nm, and the EC50 (maximum effective extract concentration eliciting 50% of maximal response) value was calculated.

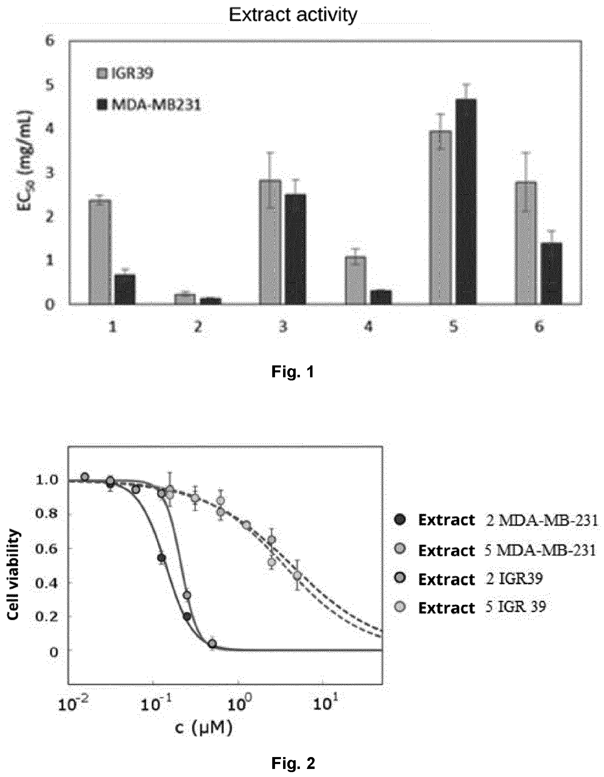

**Results.** All tested dry extracts of water-soluble polysaccharides from saffron corms have shown anti-cancer activity for human triple-negative breast cancer MDA-MB-231 and human melanoma IGR39 cells (Fig. 1).

The most active extract was #2 and had an EC50 of 0.14 µg / ml against the MDA-MB-231 cell line and 0.23 µg / ml against the IGR39 cell line. It was 17 to 34 times more active than extract #5 against the same two cell lines (Fig. 2).

## Claims

1. A process for the preparation of a water-soluble polysaccharide extract from *Crocus sativus* corms, **characterized in that** said process comprises the following steps:
(a) sorting, crushing and macerating raw *Crocus sativus* corms in room temperature water;
(b) extracting the water-soluble polysaccharides in water;
(c) precipitating water-soluble polysaccharides;
(d) drying the polysaccharide extract until the precipitated materials reach a constant weight;
(e) comminuting the polysaccharide extract to a powder;
wherein prepared water-soluble polysaccharide extract substantially comprises the polysaccharides.

2. The process for the preparation of the water-soluble polysaccharide extract from *Crocus sativus* corms according to claim 1, **characterized in that** the maceration of step (a) is performed at room temperature three times for 24 hours.

3. The process for the preparation of the water-soluble polysaccharide extract from *Crocus sativus* corms according to claim 1, **characterized in that** the ratio of crushed *Crocus sativus* corms to water is 1 g: 4 ml.

4. The process for the preparation of the water-soluble polysaccharide extract from *Crocus sativus* corms according to claim 1, **characterized in that** the extraction of step (b) repeated 2 times is sufficient for maximum extraction of water-soluble polysaccharides from saffron corns.

5. The process for the preparation of the water-soluble polysaccharide extract from *Crocus sativus* corms according to claim 1, **characterized in that** ethyl alcohol is used for the precipitation of water-soluble polysaccharides in step (c).

6. A water-soluble polysaccharide extract substantially comprising polysaccharides according to claims 1-5 for use in the prophylaxis or treatment of cancer.

7. The water-soluble polysaccharide extract for use according to claim 6, **characterized in that** the cancer is triple-negative breast cancer.

8. The water-soluble polysaccharide extract for use according to claim 6, **characterized in that** the cancer is melanoma.
